# EUROPEAN PATENT APPLICATION

(11) **EP 4 616 814 A1**
(43) Date of publication of application: **17.09.2025**
(21) Application number: 23887656.9
(22) Date of filing: 18.09.2023
(51) Int. Cl.: A61B 17/16

(54) **MEDICAL ULTRASONIC SCALPEL, MEDICAL ULTRASONIC SCALPEL SYSTEM, AND ROBOT-ASSISTED ULTRASONIC SCALPEL SYSTEM**

(30) Priority: 11.11.2022 CN 202211420752; 11.11.2022 CN 202223012938 U
(71) Applicant: SMTP Medical Co., Ltd., Beijing 102629 (CN)
(72) Inventor: CAO, Qun, Beijing 102629 (CN); ZHAN, Songtao, Beijing 102629 (CN); FANG, Yingfei, Beijing 102629 (CN)
(74) Representative: Reddie & Grose LLP
(86) International application number: PCT/CN2023/119429
(87) International publication number: WO 2024/098962

(57) **Abstract**

Disclosed are a medical ultrasonic scalpel, a medical ultrasonic scalpel system, and a robot-assisted ultrasonic scalpel system, relating to the technical field of medical instruments. The medical ultrasonic scalpel includes a blade (10) having a first end face (101) and a cutting groove (102), where the cutting groove (102) passes through the first end face (101), and the cutting groove (102) allows the first end face (101) to form at least one annular cutting face (103). During the cutting or fragmentation of bone tissue, the bone tissue corresponding to a position where the cutting groove (102) is located does not contact the cutting face (103), that is, it is not needed to cut or fragment the bone tissue corresponding to the position, but the bone tissue corresponding to the periphery of the cutting groove (102) may contact the cutting face (103) so as to be cut or fragmented. In this way, the cutting face (103) creates a relatively small area of cutting or fragmentation on the bone tissue, but can still cut or fragment the bone tissue into large pieces of bone. Additionally, since the cutting face (103) creates a smaller area of cutting or fragmentation on the bone tissue, cutting or fragmenting the bone tissue into bone pieces of the same size takes less time, achieving higher working efficiency.

## Description

### CROSS REFERENCE

The present application refers to Chinese Patent Application No. 202211420752.4 entitled "MEDICAL ULTRASONIC SCALPEL, MEDICAL ULTRASONIC SCALPEL SYSTEM, AND ROBOT-ASSISTED ULTRASONIC SCALPEL SYSTEM", and Chinese Patent Application No. 202223012938.4 entitled "MEDICAL ULTRASONIC SCALPEL, MEDICAL ULTRASONIC SCALPEL SYSTEM, AND ROBOT-ASSISTED ULTRASONIC SCALPEL SYSTEM", both of which are filed on November 11, 2022, and are incorporated herein by reference in their entireties.

### TECHNICAL FIELD

The present application relates to the technical field of medical instruments, and in particular to a medical ultrasonic scalpel, a medical ultrasonic scalpel system and a robot-assisted ultrasonic scalpel system.

### BACKGROUND ART

With the development of ultrasound technology and its integration with modern medicine, medical ultrasonic scalpels are increasingly being utilized in surgical procedures. For example, bone tissue can be cut, drilled or ground with a medical ultrasonic scalpel, thus achieving the purpose of treating the bone tissue.

In the related art, when bone tissue is to be cut or fragmented, the bone tissue is ground or drilled using a medical ultrasonic scalpel, and the bone tissue is ground or processed into smaller pieces of bone, to enable the handling of the bone tissue.

However, in the related art, the bone tissue is processed slowly and less efficiently.

### SUMMARY

The present application is intended to solve at least one of the technical problems existing in the background art. To this end, it is an objective of the present application to provide a medical ultrasonic scalpel, a medical ultrasonic scalpel system and a robot-assisted ultrasonic scalpel system, in order to alleviate the problem of low working efficiency of the medical ultrasonic scalpel in the related art.

In a first aspect, an embodiment of the present application provides a medical ultrasonic scalpel, including: a blade having a first end face and a cutting groove, where the cutting groove passes through the first end face, and the cutting groove allows the first end face to form at least one annular cutting face.

In some embodiments, the blade also has a second end face and a flank face, the first end face being opposite the second end face, the flank face connecting the first end face and the second end face, and the cutting groove being a through hole, with one end of which passes through the first end face and the other end of which passes through one of the second end face and the flank face.

In some embodiments, the blade has at least two through holes, the other end of the through hole passing through the flank face, and the through hole allowing the flank face to form a chip ejection portion.

In some embodiments, the first end face has an area larger than the area of the second end face, and the other end of the through hole passes through a part of the flank face close to the second end face.

In some embodiments, a distance L1 between axes of any two through holes in a direction X from the first end face to the second end face remains the same at first and then gradually increases.

In some embodiments, a junction of the through hole and the flank face forms a scraping portion.

In some embodiments, the scraping portion is serrated; or the scraping portion has a chamfer.

In some embodiments, the blade has a through hole, the other end of the through hole passing through the second end face.

In some embodiments, the cutting face is serrated; or the cutting face is a file surface; or an edge of the cutting face has a chamfer.

In some embodiments, an orthographic projection of the cutting groove on the first end face includes at least one of a circle, an ellipse, a rectangle and a rounded rectangle.

In some embodiments, the medical ultrasonic scalpel further includes: a bit bar connected to an end of the blade away from the first end face.

In some embodiments, the medical ultrasonic scalpel further includes: a bit body, the bit body connecting the bit bar and the blade, and in a direction parallel to the first end face, the bit body having a cross-sectional area smaller than the area of the first end face.

In a second aspect, an embodiment of the present application provides a medical ultrasonic scalpel system, including a vibration source and a medical ultrasonic scalpel of any one of the embodiments described above, where the vibration source is connected to a blade of the medical ultrasonic scalpel, and is configured to generate vibration.

In a third aspect, an embodiment of the present application provides a robot-assisted ultrasonic scalpel system including a robot-assisted surgical device and the medical ultrasonic scalpel system of the embodiment described above, where the robot-assisted surgical device is connected to a medical ultrasonic scalpel of the medical ultrasonic scalpel system to control movement of the medical ultrasonic scalpel.

A cutting groove is arranged in a blade of a medical ultrasonic scalpel according to an embodiment of the present application, the cutting groove forms an annular cutting face through a first end face, the cutting face cuts or fragments bone tissue, and the middle of the cutting face is hollow. When the bone tissue is to be cut or fragmented, the bone tissue corresponding to a position (i.e., the middle of cutting face) where the cutting groove is located does not contact the cutting face, i.e., it does not have to be cut or fragmented, but the bone tissue corresponding to the periphery of the cutting groove (i.e., the annular cutting face) may contact the cutting face to be cut or fragmented. In this way, the cutting face creates a small area of cutting on the bone tissue, but still can cut or fragment the bone tissue into large pieces of bone, and due to a small area of cutting or fragmentation on the bone tissue by the cutting face, the medical ultrasonic scalpel according to the embodiment of this application, when cutting or fragmenting the bone tissue into bone pieces of the same size, achieves reduced time and improved working efficiency.

### BRIEF DESCRIPTION OF THE DRAWINGS

In the accompanying drawings, the same reference signs denote the same or similar components or elements throughout a plurality of accompanying drawings unless otherwise specified. These accompanying drawings are not necessarily drawn to scale. It should be understood that these accompanying drawings depict only some embodiments according to the present application herein and are not to be construed as limiting the scope of the present application.
Fig. 1 is a schematic structural diagram of a medical ultrasonic scalpel according to an embodiment of the present application;
Fig. 2 is a schematic structural diagram of a blade according to an embodiment of the present application;
Fig. 3 is a right side view of a blade according to an embodiment of the present application;
Fig. 4 is a front view of a medical ultrasonic scalpel according to an embodiment of the present application;
Fig. 5 is a cross-sectional view of the medical ultrasonic scalpel of Fig. 4 along section A-A;
Fig. 6 is a top view of the medical ultrasonic scalpel of Fig. 4;
Fig. 7 is a schematic structural diagram of a further medical ultrasonic scalpel according to an embodiment of the present application;
Fig. 8 is a schematic structural diagram of a blade according to an embodiment of the present application;
Fig. 9 is a partial enlarged view of a blade section of the medical ultrasonic scalpel of Fig. 7;
Fig. 10 is a front view of a blade section of the medical ultrasonic scalpel of Fig. 7;
Fig. 11 is a top view of a blade section of the medical ultrasonic scalpel of Fig. 7;
Fig. 12 is a front view of a further medical ultrasonic scalpel according to an embodiment of the present application;
Fig. 13 is a cross-sectional view of the medical ultrasonic scalpel of Fig. 12 along section B-B;
Fig. 14 is a right side view of a blade according to an embodiment of the present application;
Fig. 15 is a left side view of a blade according to an embodiment of the present application;
Fig. 16 is a schematic structural diagram of a further medical ultrasonic scalpel according to an embodiment of the present application;
Fig. 17 is a schematic structural diagram of a blade according to an embodiment of the present application;
Fig. 18 is a partial enlarged view of a blade section of the medical ultrasonic scalpel of Fig. 16;
Fig. 19 is a front view of a blade section of the medical ultrasonic scalpel of Fig. 16;
Fig. 20 is a front view of a further medical ultrasonic scalpel according to an embodiment of the present application;
Fig. 21 is a right side view of a blade according to an embodiment of the present application;
Fig. 22 is a left side view of a blade according to an embodiment of the present application;
Fig. 23 is a schematic structural diagram of a further medical ultrasonic scalpel according to an embodiment of the present application;
Fig. 24 is a schematic structural diagram of a blade according to an embodiment of the present application;
Fig. 25 is a partial enlarged view of a blade section of the medical ultrasonic scalpel of Fig. 23;
Fig. 26 is a front view of a blade section of the medical ultrasonic scalpel of Fig. 23;
Fig. 27 is a front view of a further medical ultrasonic scalpel according to an embodiment of the present application;
Fig. 28 is a right side view of a blade according to an embodiment of the present application; and
Fig. 29 is a left side view of a blade according to an embodiment of the present application.

### List of reference signs:

10. Blade; 20. Bit bar; 30. Bit body;
101. First end face; 102. Cutting groove; 103. Cutting face; 104. Second end face; 105. Flank face; 106. Chip ejection portion; 107. Scraping portion; 201. External thread.

### DETAILED DESCRIPTION OF EMBODIMENTS

Only some exemplary embodiments will be briefly described below. As can be appreciated by those skilled in the art, the described embodiments can be modified in various ways without departing from the spirit or scope of the present application. Accordingly, the accompanying drawings and the description are considered illustrative in nature rather than limited.

From the perspective of current market developments, medical ultrasonic scalpels are gradually applied in surgical operations. Moreover, due to the characteristics of ultrasound, when a medical ultrasonic scalpel comes into contact with bone tissue with a high hardness, the bone tissue is less likely to be deformed, so that cutting or fragmentation can be produced at the contact position. When the medical ultrasonic scalpel comes into contact with soft tissue, the soft tissue will be bounced or deformed under the elastic action of the soft tissue, and will vibrate slightly with the vibration of the medical ultrasonic scalpel, thereby offsetting the energy at a blade and avoiding cutting or fragmentation. Therefore, especially for surgical operations where the surgical site is located near the interface between the bone and the soft tissue, the medical ultrasonic scalpel has significant advantages in bone cutting.

The applicant notes that in the related art, when bone tissue is to be cut or fragmented using a medical ultrasonic scalpel, the bone tissue is drilled mainly using a blade of the medical ultrasonic scalpel, and the bone tissue that needs to be cut or fragmented is ground or cut into smaller pieces of bone, to enable the handling of the bone tissue. However, the bone tissue is hard, so the bone tissue is cut or fragmented slowly and less efficiently.

In order to improve the working efficiency of a medical ultrasonic scalpel, embodiments of the present application provide a medical ultrasonic scalpel including a blade having a first end face and a cutting groove, where the cutting groove passes through the first end face, and the cutting groove allows the first end face to form at least one annular cutting face. When bone tissue is to be cut or fragmented, the bone tissue corresponding to a position where the cutting groove is located does not contact the cutting face, i.e., it does not have to be cut or fragmented, but the bone tissue corresponding to the periphery of the cutting groove (i.e., the annular cutting face) may contact the cutting face to be cut or fragmented. In this way, the cutting face creates a small area of cutting on the bone tissue, but still can cut or fragment the bone tissue into large pieces of bone, and due to a small area of cutting or fragmentation on the bone tissue by the cutting face, the medical ultrasonic scalpel according to the embodiment of the present application, when cutting or fragmenting the bone tissue into bone pieces of the same size, achieves reduced time, increased speed and improved working efficiency.

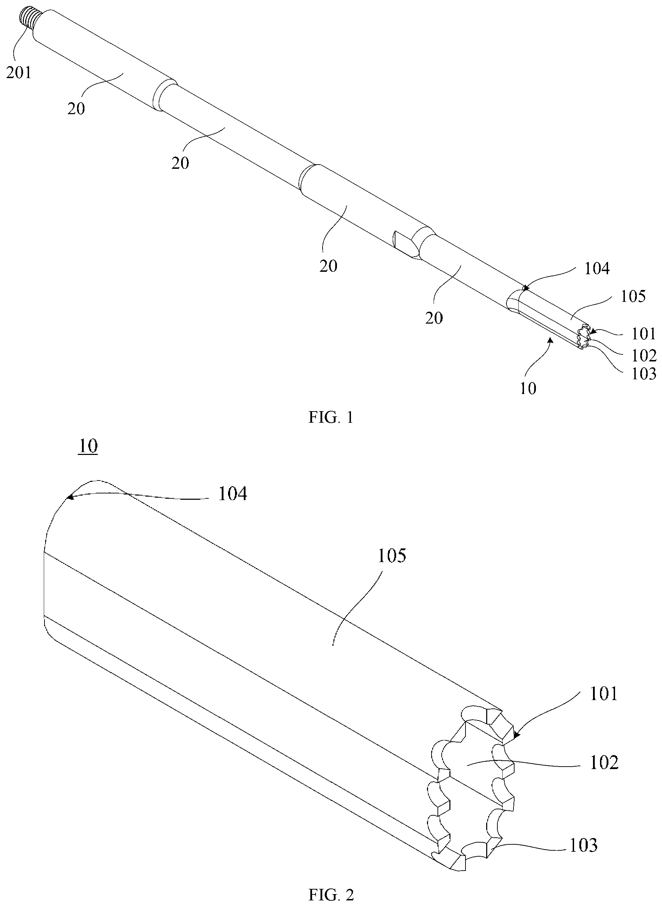

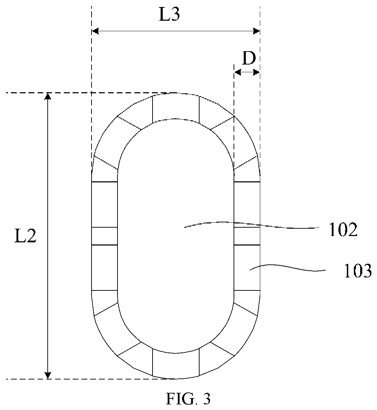

An embodiment of the present application provides a medical ultrasonic scalpel, and Fig. 1 is a schematic structural diagram of a medical ultrasonic scalpel according to an embodiment of the present application. With reference to Fig. 1, the medical ultrasonic scalpel includes a blade 10. Fig. 2 is a schematic structural diagram of a blade according to an embodiment of the present application, and Fig. 3 is a right side view of a blade according to an embodiment of the present application. With reference to Figs. 2 and 3, the blade 10 has a first end face 101 and a cutting groove 102, where the cutting groove 102 passes through the first end face 101, and the cutting groove 102 allows the first end face 101 to form at least one annular cutting face 103.

In an embodiment of the present application, the cutting face 103 is configured to cut or fragment the bone tissue. In practical applications, vibration of the blade 10 drives the cutting face 103 to vibrate, and the bone tissue is cut or fragmented during vibration of the cutting face 103.

In an embodiment of the present application, the cutting groove 102 is arranged in the blade 10, the cutting groove 102 forms an annular cutting face 103 through the first end face 101, the cutting face 103 cuts or fragments the bone tissue, and the middle of the cutting face 103 is hollow. When the bone tissue is to be cut or fragmented, the bone tissue corresponding to a position (i.e., the middle of cutting face 103) where the cutting groove 102 is located does not contact the cutting face 103, i.e., it does not have to be cut or fragmented, but the bone tissue corresponding to the periphery of the cutting groove 102 (i.e., the annular cutting face 103) may contact the cutting face 103 to be cut or fragmented. In this way, the cutting face 103 creates a small area of cutting or fragmentation on the bone tissue, but still can cut or fragment the bone tissue into large pieces of bone, and due to a small area of cutting or fragmentation on the bone tissue by the cutting face 103, the medical ultrasonic scalpel according to the embodiment of the present application, when cutting or fragmenting the bone tissue into bone pieces of the same size, achieves reduced time, increased speed and improved working efficiency.

The medical ultrasonic scalpel according to the embodiment of the present application has a large cutting face 103, which allows for wide groove cutting or fragmentation on the bone tissue.

In an implementation of the present application, the cutting face 103 is serrated, which increases the friction of the cutting face 103, so that the medical ultrasonic scalpel is stable and does not slip when it is used.

In a further implementation of the present application, if the cutting face 103 is a file surface, the friction of the cutting face 103 can also be increased, while the cutting face 103 of the file surface cuts or fragments the bone tissue faster, improving the working efficiency of the medical ultrasonic scalpel.

In a further implementation of the present application, an edge of the cutting face 103 has a chamfer.

In an implementation of the present application, a ring width D of the annular cutting face 103 is greater than or equal to 0.3 millimeters (mm) and less than or equal to 0.6 millimeters, for example, the ring width D of the annular cutting face 103 is equal to 0.45 millimeters. The strength of the cutting face 103 is guaranteed, and the cutting face 103 is also prevented from being too large and making the medical ultrasonic scalpel cumbersome to operate.

According to some embodiments of the present application, with reference to Figs. 2 and 3, the blade 10 also has a second end face 104 and a flank face 105, the first end face 101 being opposite the second end face 104, and the flank face 105 connecting the first end face 101 and the second end face 104.

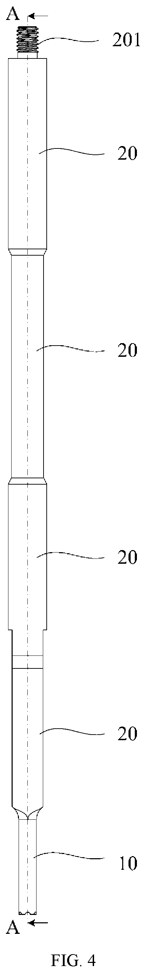

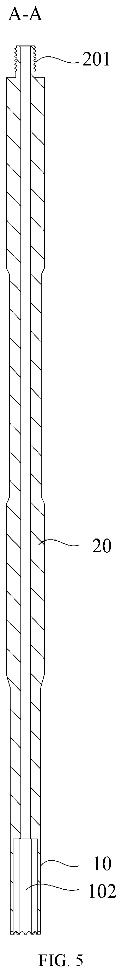

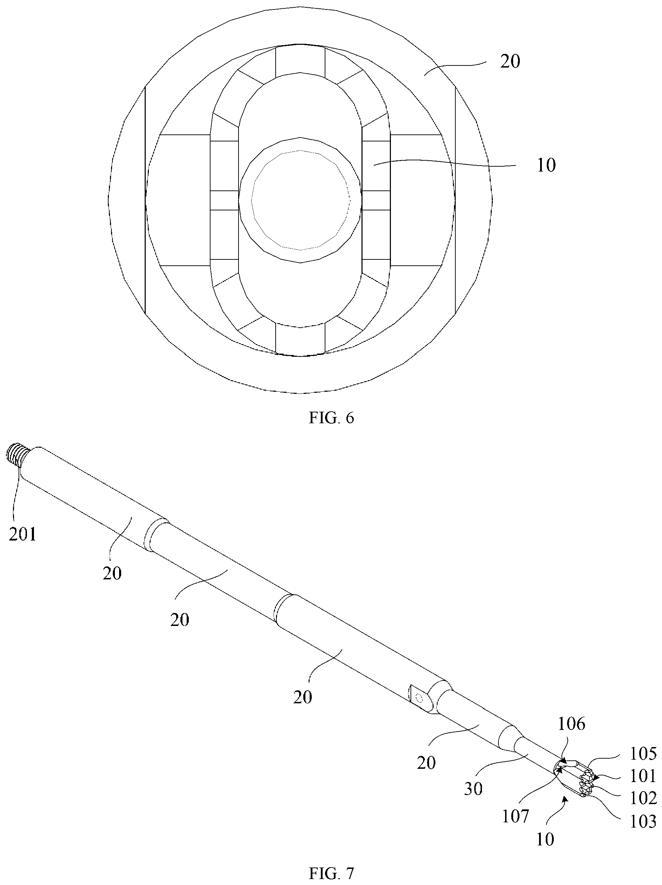

Fig. 4 is a front view of a medical ultrasonic scalpel according to an embodiment of the present application, Fig. 5 is a cross-sectional view of the medical ultrasonic scalpel of Fig. 4 along section A-A, and Fig. 6 is a top view of the medical ultrasonic scalpel of Fig. 4. With reference to Figs. 2 to 6, the cutting groove 102 is a through hole, one end of which passes through the first end face 101 and the other end of which passes through the second end face 104.

After the bone tissue is cut or fragmented, the cut bone piece enters the cutting groove 102 from the first end face 101. In an embodiment of the present application, the cutting groove 102 is arranged as a through hole, and after the bone tissue has been cut or fragmented, a tool can be used to push the bone piece in the through hole out of the second end face 104 to facilitate cleaning the blade.

In another embodiment of the present application, the cutting groove 102 may not be a through hole, i.e., the cutting groove 102 passes only through the first end face 101, and after the bone tissue is cut or fragmented, the bone piece in the cutting groove 102 can be extracted from the first end face 101 by using the tool.

In the blade 10 shown in Figs. 1 to 6, the annular cutting face 103 resembles the shape of a racetrack, i.e., the middle of the cutting face 103 is in the shape of a rectangle, with two ends of the rectangle each connected to a semicircle.

Illustratively, a length L2 of the cutting face 103 in the shape of a racetrack is greater than or equal to 4 mm and less than or equal to 6 mm, and a width L3 of the cutting face 103 in the shape of a racetrack is greater than or equal to 2 mm and less than or equal to 4 mm.

For example, the length L2 of the cutting face 103 in the shape of a racetrack is equal to 5 mm, and the width L3 of the cutting face 103 in the shape of a racetrack is equal to 2.9 mm.

In the blade 10 shown in Figs. 1 to 6, the blade 10 has a through hole.

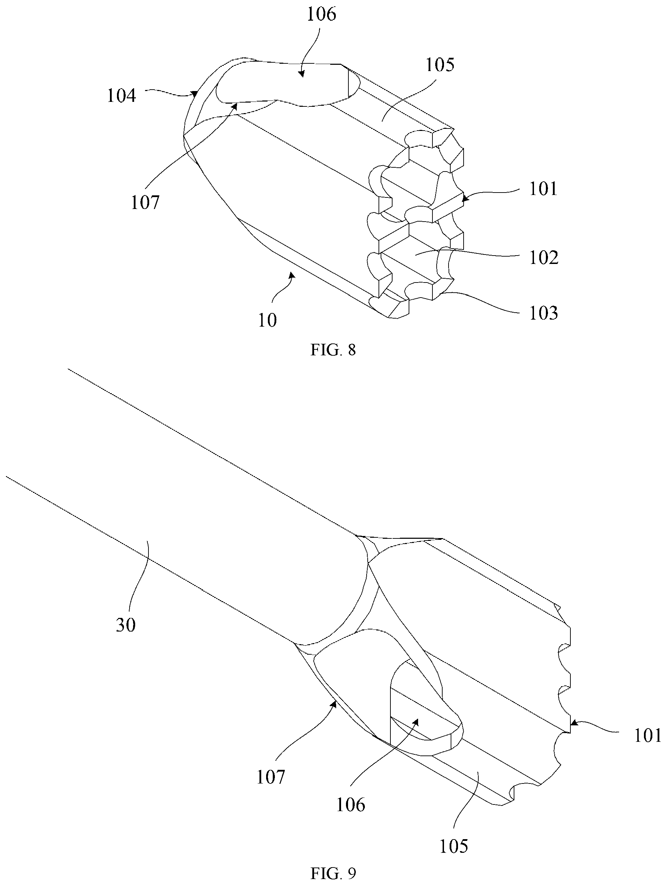

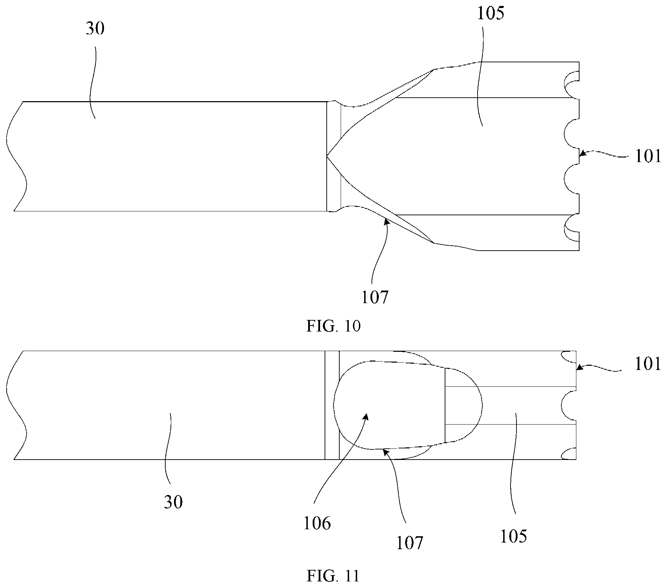

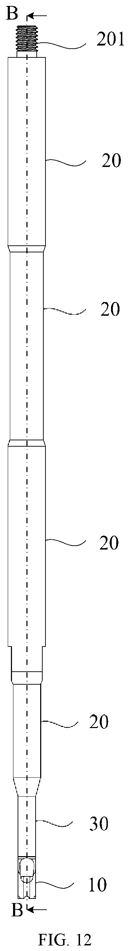

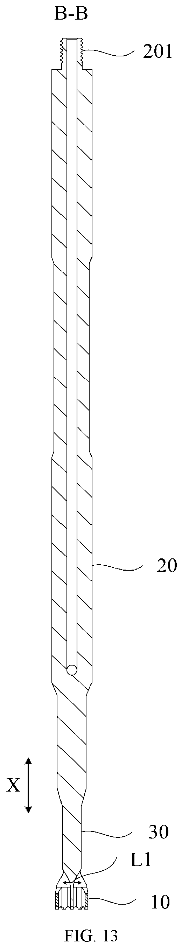

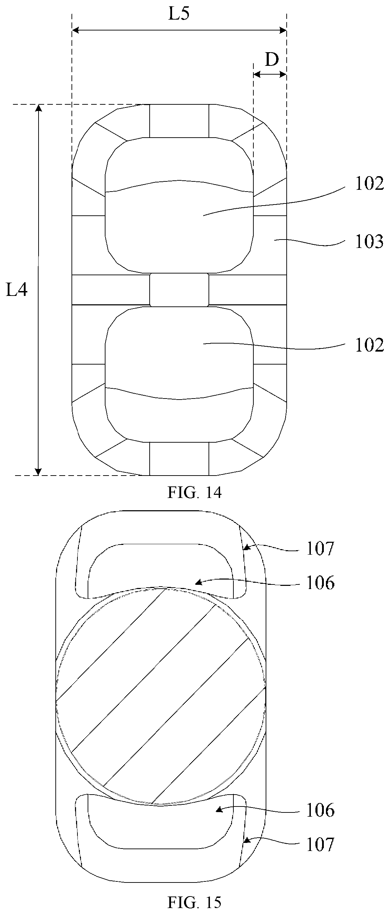

Fig. 7 is a schematic structural diagram of a further medical ultrasonic scalpel according to an embodiment of the present application. Fig. 8 is a schematic structural diagram of a blade according to an embodiment of the present application. Fig. 9 is a partial enlarged view of a blade section of the medical ultrasonic scalpel of Fig. 7. Fig. 10 is a front view of a blade section of the medical ultrasonic scalpel of Fig. 7. Fig. 11 is a top view of a blade section of the medical ultrasonic scalpel of Fig. 7. Fig. 12 is a front view of a further medical ultrasonic scalpel according to an embodiment of the present application. Fig. 13 is a cross-sectional view of the medical ultrasonic scalpel of Fig. 12 along section B-B. Fig. 14 is a right side view of a blade according to an embodiment of the present application. Fig. 15 is a left side view of a blade according to an embodiment of the present application.

With reference to Figs. 7 to 15, the blade 10 has two through holes. The other end of the through hole passes through the flank face 105, and the through hole allows the flank face 105 to form a chip ejection portion 106.

Since the cut or fragmented bone piece will enter into the through hole, in the embodiment of the present application, the other end of the through hole passes through the flank face 105, i.e., neither end of the through hole is obscured. The bone piece thus cut or fragmented may be discharged through the chip ejection portion 106, to achieve automatic cleaning of the bone piece, which is more convenient to use.

In the blade 10 shown in Figs. 7 to 15, the annular cutting face 103 is a rounded rectangle. Illustratively, the length L4 of the cutting face 103 of the rounded rectangle is greater than or equal to 4 mm and less than or equal to 6 mm, and the width L5 of the cutting face 103 of the rounded rectangle is greater than or equal to 2 mm and less than or equal to 4 mm.

For example, the length L4 of the cutting face 103 of the rounded rectangle is equal to 5 mm, and the width L5 of the cutting face 103 of the rounded rectangle is equal to 2.9 mm.

In an embodiment of the present application, the first end face 101 has an area larger than the area of the second end face 104, and the other end of the through hole passes through a part of the flank face 105 close to the second end face 104.

In the embodiment of the present application, since the area of the first end face 101 is larger than the area of the second end face 104, and the cutting face 103 is located at the first end face 101, when the bone tissue is drilled using the medical ultrasonic scalpel according to the embodiment of the present disclosure, drilling can continue even if a drilling depth exceeds the height of the blade 10 until the required drilling depth is reached.

In the embodiment of the present application, a junction of the through hole and the flank face 105 forms a scraping portion 107, that is, the scraping portion 107 is a part of the flank face 105. When a medical ultrasonic scalpel is used, it may be necessary to scrape the bone tissue, and the medical ultrasonic scalpel according to the embodiment of the present application may be more convenient to scrape the bone tissue through the scraping portion 107.

In an implementation of the present application, the scraping portion 107 is serrated, which increases the friction of the scraping portion 107, so that the medical ultrasonic scalpel is stable and does not slip when it is used.

In a further implementation of the present application, the scraping portion 107 has a chamfer.

In some embodiments of the present application, with reference to Fig. 13, a distance L1 between axes of any two through holes in a direction X from the first end face 101 to the second end face 104 remains the same at first and then gradually increases.

Exemplarily, the distance L1 between the axes of any two through holes is constant from the first end face 101 to the middle of the first end face 101 and the second end face 104, and the distance L1 between the axes of any two through holes gradually increases from the middle of the first end face 101 and the second end face 104 to the second end face 104.

In the embodiment of the present application, the end of the through hole away from the first end face 101 is proximal to the flank face 105, that is, a part of the through hole close to the second end face 104 is inclined outwardly, and the bone piece within the through hole is also inclined outwardly when moved, so that the bone piece within the through hole is easier to drain.

In the blade 10 shown in Figs. 7 to 15, the blade 10 has two through holes.

The two through holes form two annular cutting faces 103 at the first end face 101.

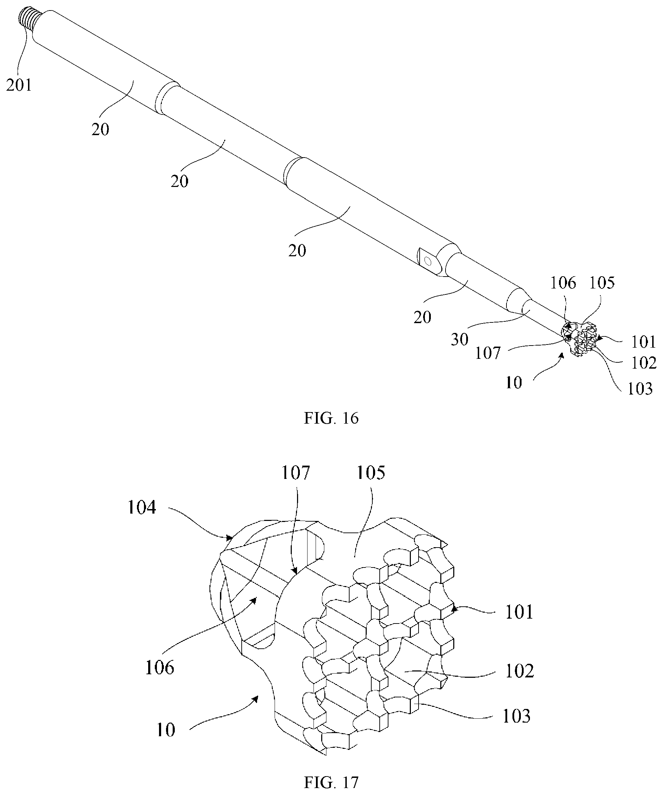

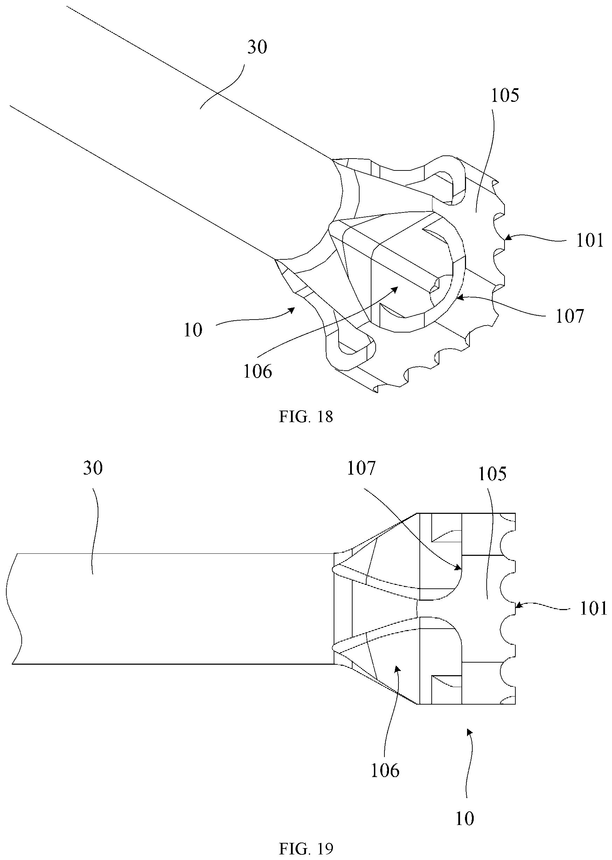

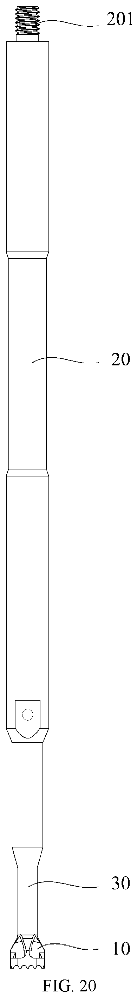

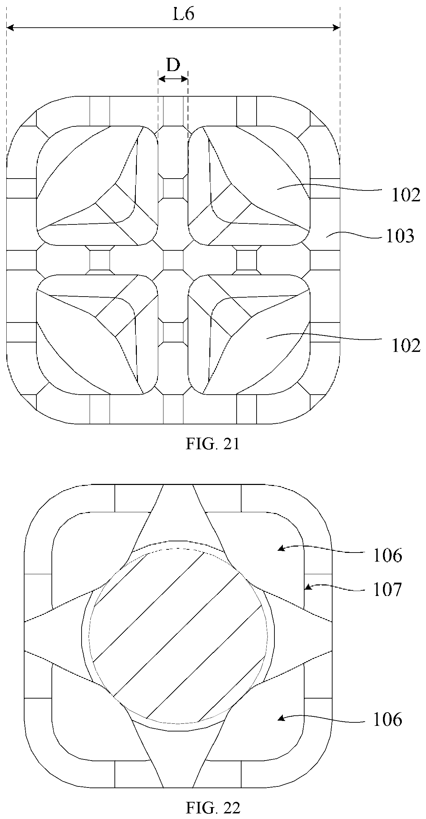

Fig. 16 is a schematic structural diagram of a further medical ultrasonic scalpel according to an embodiment of the present application. Fig. 17 is a schematic structural diagram of a blade according to an embodiment of the present application. Fig. 18 is a partial enlarged view of a blade section of the medical ultrasonic scalpel of Fig. 16. Fig. 19 is a front view of a blade section of the medical ultrasonic scalpel of Fig. 16. Fig. 20 is a front view of a further medical ultrasonic scalpel according to an embodiment of the present application. Fig. 21 is a right side view of a blade according to an embodiment of the present application. Fig. 22 is a left side view of a blade according to an embodiment of the present application.

With reference to Figs. 16 to 22, the blade 10 has four through holes, which form four annular cutting faces 103 on the first end face 101.

In other implementations, the blade 10 may have three through holes, five through holes, or more through holes, which is not limited in the present application. A plurality of through holes may be distributed symmetrically around a central axis of the blade 10.

In the blade 10 shown in Figs. 16 to 22, the annular cutting face 103 has a rounded square shape. Illustratively, the length L6 of a side of the cutting face 103 of the rounded square is greater than or equal to 4 mm and less than or equal to 6 mm. For example, the length L6 of the side of the cutting face 103 of the rounded square is equal to 5 mm.

In the blade 10 shown in Figs. 7 to 22, the annular cutting face 103 is polygonal.

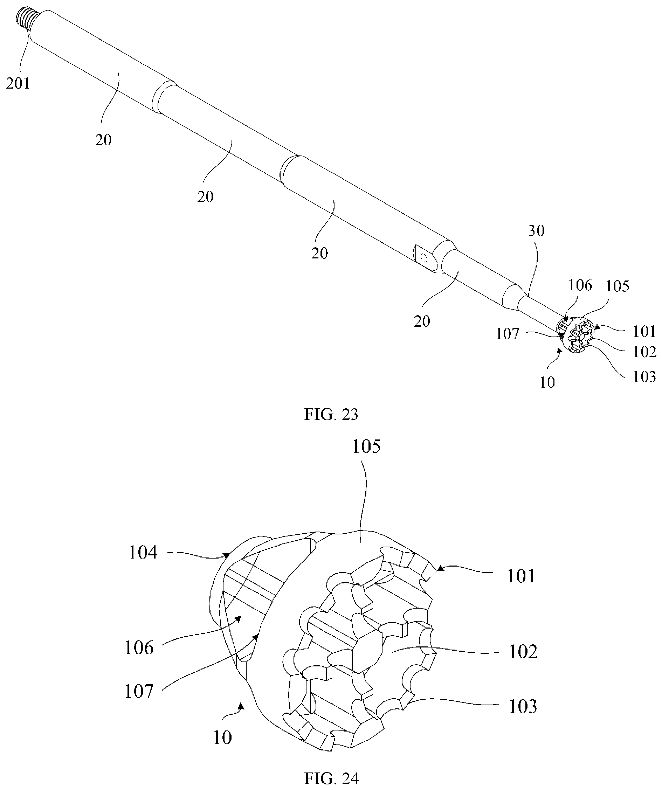

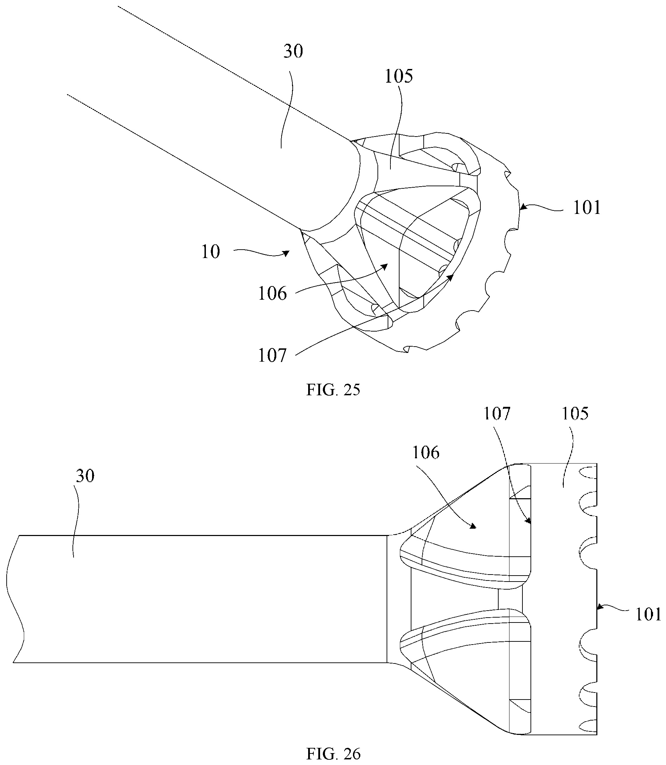

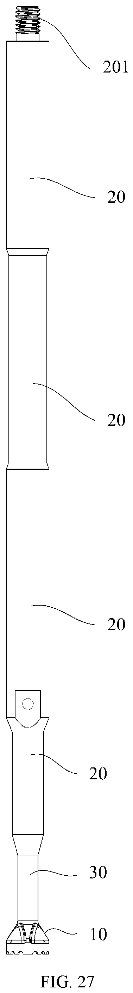

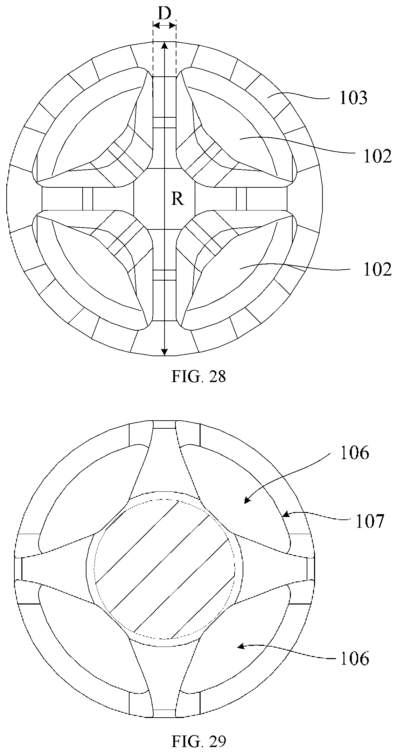

Fig. 23 is a schematic structural diagram of a further medical ultrasonic scalpel according to an embodiment of the present application. Fig. 24 is a schematic structural diagram of a blade according to an embodiment of the present application. Fig. 25 is a partial enlarged view of a blade section of the medical ultrasonic scalpel of Fig. 23. Fig. 26 is a front view of a blade section of the medical ultrasonic scalpel of Fig. 23. Fig. 27 is a front view of a further medical ultrasonic scalpel according to an embodiment of the present application. Fig. 28 is a right side view of a blade according to an embodiment of the present application. Fig. 29 is a left side view of a blade according to an embodiment of the present application.

With reference to Figs. 23 to 29, the annular cutting face 103 is circular. Illustratively, the diameter R of the circular cutting face 103 is greater than or equal to 4 mm and less than or equal to 8 mm. For example, the diameter R of the circular cutting face 103 is equal to 6.2 mm.

The description of the cutting face 103 in the above embodiments is a description of the overall shape of the cutting face 103.

In the embodiment of the present application, the shape of an orthographic projection of the cutting groove 102 on the first end face 101 is not limited, and may include at least one of a circle, an ellipse, a rectangle and a rounded rectangle.

In a further implementation, the shape of the orthographic projection of the cutting groove 102 on the first end face 101 may also be an irregular shape.

In some embodiments of the present application, with reference to Figs. 7, 12, 13, 16, 20, 23 and 27, the medical ultrasonic scalpel further includes a bit bar 20, the bit bar 20 being connected to an end of the blade 10 away from the first end face 101.

In the embodiment of the present application, the bit bar 20 may be used for connection to other device, facilitating connection of the medical ultrasonic scalpel to other device. Illustratively, said other device may be a vibration source.

Illustratively, an end of the bit bar 20 away from the blade 10 is connected with an external thread 201, and the bit bar 20 may be connected to other device by means of a thread.

In some embodiments of the present application, with reference to Figs. 7, 12, 13, 16, 20, 23 and 27, the medical ultrasonic scalpel further includes a bit body 30, the bit body 30 connects the bit bar 20 and the blade 10, and in a direction parallel to the first end face 101, the bit body 30 has a cross-sectional area smaller than the area of the first end face 101.

In the embodiment of the present application, since the cross-sectional area of the bit body 30 is smaller than the area of the first end face 101, and the cutting face 103 is located at the first end face 101, when the bone tissue is drilled using the medical ultrasonic scalpel according to the embodiment of the present disclosure, drilling can continue even if a drilling depth exceeds the height of the blade 10 until the required drilling depth is reached. At the same time, the bit body 30 is connected to the vibration source and the blade 10, and the size of the bit body 30 is small to adjust the vibration of the blade 10 through the bit body 30.

In an implementation of the embodiment of the present application, a central axis of the cutting groove 102 is parallel to a central axis of the bit body 30, reducing resistance when the medical ultrasonic scalpel is used.

In the embodiment of the present application, the second end face 104 of the blade 10 is connected to the bit body 30, at which point the second end face 104 of the blade 10 is covered by the bit body 30.

In a further implementation, the bit body 30 and the blade 10 may be integrally formed.

An embodiment of the present application further provides a medical ultrasonic scalpel system including a vibration source and the medical ultrasonic scalpel of the embodiment described above, the vibration source being connected to the blade 10 of the medical ultrasonic scalpel, and the vibration source being configured to generate vibration.

The medical ultrasonic scalpel system according to the embodiment of the present application has a high working efficiency.

An embodiment of the present application also provides a robot-assisted ultrasonic scalpel system. The robot-assisted ultrasonic scalpel system includes a robot-assisted surgical device and the medical ultrasonic scalpel system of the embodiment described above. The robot-assisted surgical device is connected to a medical ultrasonic scalpel of the medical ultrasonic scalpel system to control movement of the medical ultrasonic scalpel.

A robot-assisted ultrasound osteodynamic system according to an embodiment of the present application improves the working efficiency of orthopedic surgery.

It should be understood that, in this description, the orientations or positional relationships or dimensions denoted by the terms, such as "center", "longitudinal", "transverse", "length", "width", "thickness", "upper", "lower", "front", "rear", "left", "right", "vertical", "horizontal", "top", "bottom", "inner", "outer", "clockwise", "counterclockwise", "axial", "radial" and "circumferential", are the orientations or positional relationships or dimensions shown on the basis of the drawings, and these terms are used merely for ease of description, rather than indicating or implying that the device or element referred to must have particular orientations and be constructed and operated in the particular orientations, and therefore should not be construed as limiting the scope of protection of the present application.

In addition, the terms such as "first", "second" and "third" are merely for descriptive purposes and should not be construed as indicating or implying relative importance or implicitly indicating the number of technical features indicated. Thus, the features defined with "first", "second" and "third" may explicitly or implicitly include one or more features. In the description of the present application, the term "plurality of" means two or more, unless specifically and specifically limited otherwise.

In the present application, unless expressly stated or limited otherwise, the terms such as "mounting", "connection", "connected" and "fixing" should be interpreted broadly, for example, either fixed or detachable connection, or integration; may be a mechanical connection, or an electrical connection, or communication; and may be a direct connection or an indirect connection by means of an intermediate medium, or may be internal communication between two elements or interaction between the two elements. For those of ordinary skills in the art, the specific meaning of the terms mentioned above in the present application may be construed according to specific circumstances.

In the present application, unless expressly stated or limited otherwise, the expression of the first feature being "above" or "below" the second feature may include the case where the first feature is in direct contact with the second feature, and may also include the case where the first and second features are not in direct contact but are contacted via another feature therebetween. Furthermore, the first feature being "over", "above" or "on" the second feature includes the case where the first feature is directly or obliquely above the second feature, or merely indicates that the first feature is at a higher level than the second feature. The first feature being "below", "under" or "beneath" the second feature includes the case where the first feature is directly or obliquely below the second feature, or merely indicates that the first feature is at a lower level than the second feature.

This description provides many different embodiments or examples that can be used to implement the present application. It should be understood that these various embodiments or examples are purely illustrative and are not intended to limit the scope of protection of the present application in any way. On the basis of the disclosure of the description of the present application, those skilled in the art will be able to conceive of various changes or substitutions. Any changes or substitutions shall fall within the scope of protection of the present application. Therefore, the scope of protection of the present application shall be subject to the scope of protection of the claims.

## Claims

1. A medical ultrasonic scalpel, comprising:
a blade (10) having a first end face (101) and a cutting groove (102), wherein the cutting groove (102) passes through the first end face (101), and the cutting groove (102) allows the first end face (101) to form at least one annular cutting face (103).

2. The medical ultrasonic scalpel according to claim 1, wherein the blade (10) also has a second end face (104) and a flank face (105), the first end face (101) being opposite the second end face (104), the flank face (105) connecting the first end face (101) and the second end face (104), and the cutting groove (102) being a through hole, with one end of which passes through the first end face (101) and the other end of which passes through one of the second end face (104) and the flank face (105).

3. The medical ultrasonic scalpel according to claim 2, wherein the blade (10) has at least two through holes, the other end of the through hole passing through the flank face (105), and the through hole allowing the flank face (105) to form a chip ejection portion (106).

4. The medical ultrasonic scalpel according to claim 3, wherein the first end face (101) has an area larger than the area of the second end face (104), and the other end of the through hole passes through a part of the flank face (105) close to the second end face (104).

5. The medical ultrasonic scalpel according to claim 3, wherein in a direction (X) from the first end face (101) to the second end face (104), a distance L1 between axes of any two through holes remains the same at first and then gradually increases.

6. The medical ultrasonic scalpel according to claim 3, wherein a junction of the through hole and the flank face (105) forms a scraping portion (107).

7. The medical ultrasonic scalpel according to claim 6, wherein the scraping portion (107) is serrated; or the scraping portion (107) has a chamfer.

8. The medical ultrasonic scalpel according to claim 2, wherein the blade (10) has one through hole, the other end of which passes through the second end face (104).

9. The medical ultrasonic scalpel according to any one of claims 1 to 8, wherein the cutting face (103) is serrated; or the cutting face (103) is a file surface; or an edge of the cutting face (103) has a chamfer.

10. The medical ultrasonic scalpel according to any one of claims 1 to 8, wherein an orthographic projection of the cutting groove (102) on the first end face (101) comprises at least one of a circle, an ellipse, a rectangle and a rounded rectangle.

11. The medical ultrasonic scalpel according to claim 10, wherein the medical ultrasonic scalpel further comprises:
a bit bar (20) connected to an end of the blade (10) away from the first end face (101).

12. The medical ultrasonic scalpel according to claim 11, wherein the medical ultrasonic scalpel further comprises:
a bit body (30), the bit body (30) connecting the bit bar (20) and the blade (10), and in a direction parallel to the first end face (101), the bit body (30) having a cross-sectional area smaller than the area of the first end face (101).

13. A medical ultrasonic scalpel system, comprising a vibration source and a medical ultrasonic scalpel of any one of claims 1 to 12, wherein
the vibration source is connected to a blade (10) of the medical ultrasonic scalpel, and is configured to generate vibration.

14. A robot-assisted ultrasonic scalpel system, comprising a robot-assisted surgical device and a medical ultrasonic scalpel system of claim 13, wherein
the robot-assisted surgical device is connected to a medical ultrasonic scalpel of the medical ultrasonic scalpel system to control movement of the medical ultrasonic scalpel.
